# EUROPEAN PATENT APPLICATION

(11) **EP 4 834 871 A1**
(43) Date of publication of application: **04.11.2026**
(21) Application number: 24912628.5
(22) Date of filing: 19.12.2024
(51) Int. Cl.: C07C 29/152, C02F 1/42, C02F 3/28, C07B 61/00, C07C 31/04

(54) **METHANOL PRODUCTION METHOD AND METHANOL PRODUCTION DEVICE**

(30) Priority: 25.12.2023 JP 2023218417
(71) Applicant: MITSUBISHI GAS CHEMICAL COMPANY, INC., Chiyoda-ku Tokyo 100-8324 (JP)
(72) Inventor: SHIBATA, Akihiro, Tokyo 100-8324 (JP); ABE, Takanori, Tokyo 100-8324 (JP); KAKIMI, Atsushi, Tokyo 100-8324 (JP)
(74) Representative: Vossius & Partner Patentanwälte Rechtsanwälte mbB
(86) International application number: PCT/JP2024/045016
(87) International publication number: WO 2025/142739

(57) **Abstract**

A method for producing methanol, including a step (A) of obtaining a synthesis gas containing at least carbon dioxide and hydrogen, a step (B) of reacting the synthesis gas in the presence of a catalyst to obtain a methanol mixture, a step (C) of distilling the methanol mixture to separate methanol and distillation wastewater, respectively, a step (D) of removing an acid and/or a base and a salt thereof from the distillation wastewater, and a step (E) of feeding a gas and/or a liquid that is obtained in the step (D) to the step (A).

## Description

### Technical Field

The present invention relates to a method for producing methanol and an apparatus for producing methanol.

### Background Art

In conventional methods for industrially producing methanol using natural gas as a raw material, methanol is synthesized from a synthetic gas that is obtained by adding steam to the natural gas (main component: methane), which is a raw material, to cause steam reforming. Since a reaction product obtained by the synthesis is a methanol mixture containing a component other than methanol (hereinafter, also referred to as crude methanol), the crude methanol is purified by a distillation step, and purified methanol is obtained. In the distillation step, since a certain amount of wastewater is discharged to maintain the methanol purity, the reuse of the wastewater, such as a use for steam reforming, has been proposed in view of reducing environmental load.

For example, in Patent Literature 1, for the purpose of collecting a fusel oil and converting the fusel oil into methanol, disclosed is a method for producing methanol, in which (a) a carbonaceous raw material is reacted with a gasifying agent selected from steam, carbon dioxide, and oxygen to generate a methanol synthetic gas; (b) the synthetic gas is reacted on a methanol synthesis catalyst to collect a crude methanol liquid product from the reacted gas; (c) a hydroxide or carbonate of an alkali metal is added to the crude methanol liquid product; (d) a mixture to be obtained is distilled, and a purified methanol stream and a liquid fusel oil stream containing an organic hydrocarbon having a higher boiling point than methanol and the alkali metal compound are separated from the mixture; and (e) the fusel oil stream is recirculated into the synthetic gas generation reaction, thereby converting the organic hydrocarbon into a synthetic gas or the like.

In Patent Literature 2, for the purpose of providing a method and an apparatus in which a volatile organic component is collected from a fusel waste oil stream that is generated in a methanol synthesis plant to increase the product yield, and the waste oil stream is reduced or removed at the same time, disclosed is a methanol synthesis plant including, in addition to a predetermined configuration requirement, a fusel oil stripper that strips the volatile organic component from the fusel waste oil stream; and a recirculation line for supplying the stripped organic component to a hydrocarbon gas supply raw material that is to flow into a reforming apparatus.

In Patent Literature 3, for the purpose of using wastewater from a distillation step in a process or the like, disclosed is a method for producing methanol including, in addition to a predetermined configuration requirement, steps of, upon producing methanol from a hydrocarbon, (a) reacting the hydrocarbon with steam to generate a synthetic gas containing hydrogen, carbon monoxide, and carbon dioxide as main components, (b) reacting the synthetic gas on a methanol synthesis catalyst to collect generated crude methanol in a liquid phase from the reacted gas, and (c) distilling the collected crude methanol to separate the crude methanol into purified methanol and wastewater containing a low-boiling point organic compound, a high-boiling point compound, and an organic acid, in which, in the step (c), a neutralization step with an alkali metal is not performed, and the wastewater is brought into contact with a gas-phase hydrocarbon to humidify the hydrocarbon and is supplied to the step (a).

In Patent Literature 4, for the purpose of providing a method in which a hydrocarbon is humidified using wastewater from a distillation step to which an alkali metal salt, which is desired to be collected, has been added and steam for a process, that is, expensive boiler water, is reduced, disclosed is a method for producing methanol from a hydrocarbon, including steps of (a) reacting a hydrocarbon with steam to generate a synthetic gas containing hydrogen, carbon monoxide, and carbon dioxide as main components, (b) reacting the synthetic gas on a methanol synthesis catalyst to collect generated crude methanol in a liquid phase from a reacted gas, and (c) distilling the collected crude methanol to separate the crude methanol into purified methanol and wastewater containing a low-boiling point organic compound, a high-boiling point compound, and an organic acid, in which the hydrocarbon is humidified by being brought into contact with the wastewater on which a neutralization step has been performed with an alkali metal salt or a hydroxide in the step (c), next, further humidified by being brought into contact with condensed water that is separated from the synthetic gas in the step (a), and is supplied to the step (a).

In Patent Literature 5, for the purpose of providing a method having a high economic benefit as a whole as compared with conventional technical methods for producing methanol from organic waste, disclosed is a method for generating a biogas from organic waste in which the concentration of a biogas with a concentrating apparatus by anaerobic digestion or the like is characterized.

In Patent Literature 6, for the purpose of significantly saving capital cost and operation cost, disclosed is a system for producing a bio product in which an anaerobic digestion apparatus that converts biomass into a mixture of methane and carbon dioxide, a synthetic gas generation apparatus capable of converting methane into a mixture of carbon monoxide and hydrogen, and a gas conversion apparatus are combined.

In Patent Literature 7, for the purpose of obtaining hydrogen necessary for fixing carbon dioxide at low cost, converting the carbon dioxide into methanol using the hydrogen, and fixing the methanol, disclosed is an apparatus for producing methanol using biomass, including a methane generation portion that generates methane and carbon dioxide by the anaerobic fermentation of biomass, a methane decomposition portion that decomposes the methane generated in the methane generation portion into carbon and hydrogen using a catalyst, and a methanol production portion that reacts carbon dioxide and the hydrogen generated in the methane decomposition portion using a catalyst to synthesize methanol.

### Citation List

### Patent Literature

Patent Literature 1: Japanese Patent Publication No. 03-031694
Patent Literature 2: Japanese Patent Laid-Open No. 07-145089
Patent Literature 3: Japanese Patent Publication No. 08-002808
Patent Literature 4: Japanese Patent No. 3848716
Patent Literature 5: International Publication No. WO 2018115596
Patent Literature 6: U.S. Patent No. 10240119
Patent Literature 7: Japanese Patent Laid-Open No. 11-188262

### Summary of Invention

### Technical Problem

However, in Patent Literature 1, since it is inevitable to concentrate the alkali metal compound in water to leave a liquid containing the alkali metal compound behind, there is a risk that equipment made of a metallic material may be corroded.

In Patent Literature 2, it is not possible to remove an alkali content from the fusel oil before being brought into contact with the gas to be reformed, and there is a description of the stripper, but a corrosion risk exists in the stripper.

In Patent Literature 3, a neutralization step with an alkali metal does not exist in the distillation step, and there is no need to remove an alkali content from fusel oil; however, in a case where no alkali metals are introduced in the distillation step, impurities are likely to be insufficiently separated, and the product quality may be adversely affected.

In Patent Literature 4, it is described that neutralization with an alkali metal is performed in the distillation step, but there is no step of removing a salt generated by the neutralization. Therefore, a corrosion risk due to an alkali has been reduced, but a risk of corrosion due to the concentration of a salt still remains.

In Patent Literature 5, the contents of synthesizing methanol from organic waste are described, but there is no description regarding distillation wastewater that is discharged in the distillation step, and surely, there is no description regarding a corrosion risk at the time of recycling distillation wastewater.

In Patent Literature 6, the contents of synthesizing methanol from biomass are described, but there is no description regarding distillation wastewater that is discharged in the distillation step, and surely, there is no description of a corrosion risk at the time of recycling distillation wastewater.

In Patent Literature 7, the contents of synthesizing methanol from carbon dioxide and hydrogen using digestion gas as a raw material are described, but there is no description regarding distillation wastewater that is discharged in the distillation step, and surely, there is no description regarding a corrosion risk at the time of recycling distillation wastewater.

As described above, as a result of intensive studies, the present inventors found that wastewater may be acidic due to a component that is generated as a by-product of methanol, such as formic acid, and may become alkaline in a case where the component is neutralized with an excess amount of an alkali, and the reuse of distillation wastewater as it is for steam reforming or the like creates a risk of the corrosion of equipment for steam reforming or the like. Additionally, it was found that in the case of reusing distillation wastewater in an alkaline state, carbon dioxide that is contained in a gas that serves as a raw material for the synthesis of methanol reacts with an alkali, and the output level deteriorates.

Furthermore, as a result of intensive studies, the present inventors found that even in a case where wastewater is neutralized to be neutral, a salt that is generated by the neutralization is still likely to corrode equipment.

The present invention has been made in consideration of the above-described circumstance, and an object of the present invention is to provide a method for producing methanol using natural gas or waste as a raw material, in which corrosion of equipment due to distillation wastewater is reduced while the distillation wastewater is recycled.

### Solution to Problem

As a result of intensive studies for achieving the above-described object, the present inventors have found that the present invention having the following configuration makes it possible to reduce corrosion of equipment due to distillation wastewater while the distillation wastewater is recycled and completed the present invention. That is, the present invention is as described below.
[1] A method for producing methanol, comprising:
   a step (A) of obtaining a synthesis gas containing at least carbon dioxide and hydrogen;
   a step (B) of reacting the synthesis gas in the presence of a catalyst to obtain a methanol mixture;
   a step (C) of distilling the methanol mixture to separate methanol and distillation wastewater, respectively;
   a step (D) of removing an acid and/or a base and a salt thereof from the distillation wastewater; and
   a step (E) of feeding a gas and/or a liquid that is obtained in the step (D) to the step (A).
[2] The method for producing methanol according to [1], wherein a molar flow rate of a carbon atom that is contained in the gas and/or the liquid that is obtained in the step (D) is 70 mol% or more based on a molar flow rate of a carbon atom that is contained in the distillation wastewater.
[3] The method for producing methanol according to [1] or [2], wherein the step (A) comprises a step (F) of reforming a hydrocarbon that is contained in the gas and/or the liquid that is obtained in the step (D) to obtain a reformed gas.
[4] The method for producing methanol according to any one of [1] to [3], further comprising:
   a desulfurization step (G).
[5] The method for producing methanol according to any one of [1] to [4], wherein the step (D) comprises a step of removing the acid and/or the base and the salt thereof with an ion exchanger.
[6] The method for producing methanol according to any one of [1] to [5], wherein the step (D) comprises a step of removing the acid and/or the base and the salt thereof by an anaerobic treatment.
[7] The method for producing methanol according to any one of [1] to [6], further comprising:
   a step (H) of combusting a gas that is obtained by the anaerobic treatment to recover heat.
[8] An apparatus for producing methanol, comprising:
   a synthesis gas preparation unit;
   a methanol synthesis unit;
   a distillation unit; and
   a deionization unit or an anaerobic treatment unit,
   wherein a gas and/or a liquid that is obtained from the deionization unit or the anaerobic treatment unit is used in the synthesis gas preparation unit.
[9] The apparatus for producing methanol according to [8], wherein the synthesis gas preparation unit comprises a reformer.
[10] The apparatus for producing methanol according to [8] or [9], further comprising:
   a desulfurization unit.

### Advantageous Effect of Invention

The present invention makes it possible to provide a method for producing methanol in which corrosion of equipment due to distillation wastewater is reduced while the distillation wastewater is recycled.

### Brief Description of Drawings

[FIG. 1] FIG. 1 is a schematic view showing an example of a production apparatus that is used in a method for producing methanol of the present embodiment.
[FIG.2] FIG. 2 is a schematic view showing another example of the production apparatus that is used in the method for producing methanol of the present embodiment.
[FIG. 3] FIG. 3 is a schematic view showing one example of a production apparatus that is used in a method for producing methanol corresponding to a comparative example.
[FIG. 4] FIG. 4 is a schematic view showing another example of the production apparatus that is used in the method for producing methanol corresponding to the comparative example.
[FIG. 5] FIG. 5 is a schematic view showing still another example of the production apparatus that is used in the method for producing methanol corresponding to the comparative example.
[FIG. 6] FIG. 6 is a schematic view showing an example of an anaerobic treatment unit of the present embodiment.
[FIG. 7] FIG. 7 is a schematic view showing examples of a gasification preparation step of the present embodiment.
[FIG. 8] FIG. 8 is a schematic view showing more examples of the gasification preparation step of the present embodiment.

### Description of Embodiments

Hereinafter, an embodiment for carrying out the present invention (hereinafter, simply referred to as "the present embodiment") will be described in detail with reference to drawings as necessary, but the present invention is not limited to the following embodiment. The present invention can be modified in a variety of manners within the scope of the gist thereof. In the drawings, the same elements will be given the same reference sign, and duplicate description will be omitted. In addition, positional relationships, such as up, down, right, and left, will be based on the positional relationship shown in the drawings unless particularly otherwise described. Furthermore, dimensional ratios of the drawings are not limited to ratios shown in the drawings.

### [Method for producing methanol]

A method for producing methanol of the present embodiment is a method for producing methanol, including
a step (A) of obtaining a synthesis gas containing at least carbon dioxide and hydrogen,
a step (B) of reacting the synthesis gas in the presence of a catalyst to obtain a methanol mixture,
a step (C) of distilling the methanol mixture to separate methanol and distillation wastewater, respectively,
a step (D) of removing an acid and/or a base and a salt thereof from the distillation wastewater, and
a step (E) of feeding a gas and/or a liquid that is obtained in the step (D) to the step (A).

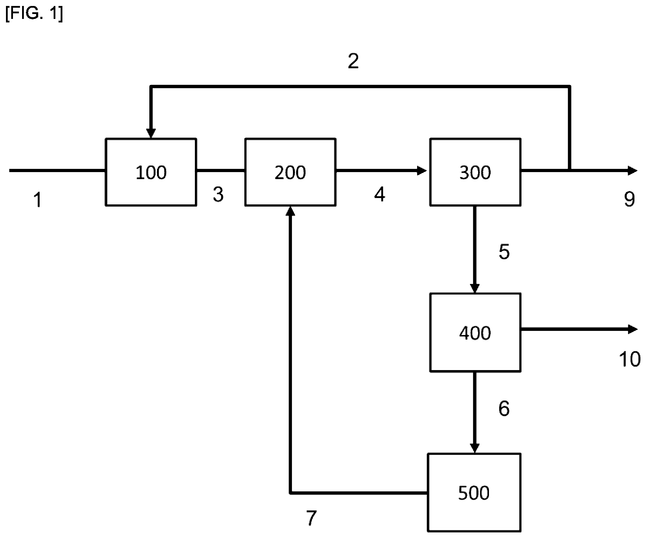

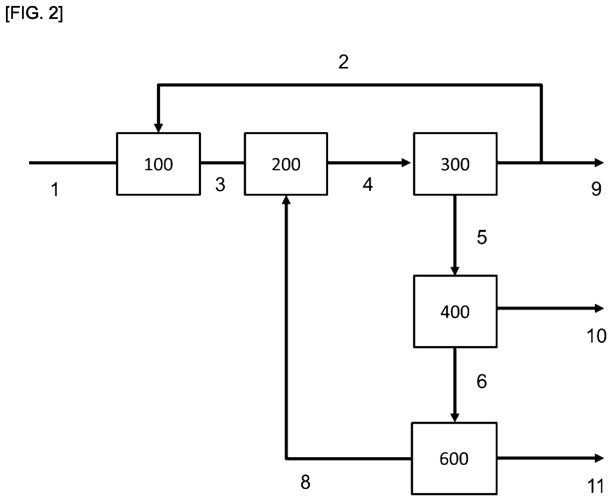

Hereinafter, the method for producing methanol of the present embodiment will be described using apparatuses for producing methanol of FIGS. 1 and 2. However, the method for producing methanol of the present embodiment is not limited to embodiments in which the apparatuses for producing methanol of FIGS. 1 and 2 are used.

### [Step (A)]

The step (A) is a step of obtaining a synthesis gas containing at least carbon dioxide and hydrogen with a synthesis gas preparation unit 200 as shown in FIGS. 1 and 2. In a case where the method for producing methanol of the present embodiment includes a desulfurization step (G) with a desulfurization unit 100, a hydrocarbon-containing gas 1 passes through the desulfurization unit 100 and turns into a desulfurized gas 3 together with a recycled gas 2 in a case where there is the recycled gas 2, then, is supplied to the synthesis gas preparation unit 200 together with a deionized distillation wastewater 7 or a digestion gas 8, and prepared into a synthesis gas 4 therein. The synthesis gas 4 is a gas that is used for the synthesis of methanol and is a gas containing hydrogen, carbon monoxide, and carbon dioxide as main components.

### [Step (F)]

From the viewpoint of improvement in methanol yield or the like, the step (A) preferably includes a reforming step of reforming the hydrocarbon-containing gas 1 with a reformer to obtain a reformed gas and more preferably includes a step (F) of reforming, particularly, the hydrocarbon-containing gas 1 that is contained in a gas and/or a liquid that is obtained in the step (D), which will be described below, to obtain a reformed gas.

A reforming method is not particularly limited, and examples thereof include steam methane reforming (SMR), autothermal reforming (ATR), two-stage reforming (SMR + ATR), and a partial oxidation method. In a case where the hydrocarbon-containing gas 1 is natural gas or naphtha, the step (A) may include a pre-reforming step of providing a pre-reformer upstream of a reforming unit and reforming the natural gas or the naphtha at approximately 500°C to produce a methane-rich gas (not particularly limited; however, for example, the CH4 content ratio is approximately 30 to 50 mol%).

As the reforming temperature, a conventionally well-known temperature can be used, there is no particular limitation, and the reforming temperature can be set to, for example, 750°C to 1000°C. In addition, a catalyst may be used for the reforming, a conventionally well-known catalyst may be used as such a catalyst, there is no particular limitation, and examples thereof include nickel-based catalysts.

The hydrocarbon-containing gas 1 is not particularly limited, and examples thereof include purified gases and fossil fuel gases. The purified gases are not particularly limited, and examples thereof include methane, ethane, propane, butane, and gas mixtures thereof. Examples of the fossil fuel gases include natural gas (NG) containing methane as a main component, liquefied petroleum gas (LPG), and naphtha. In a case where the hydrocarbon-containing gas is a fossil fuel gas, a sulfur content is contained in the gas components, and it is thus preferable to use the desulfurized gas 3 that has undergone the desulfurization step (G), which will be described below, in the step (A).

The step (A) may include a gasification preparation step of gasifying and/or combusting an organic matter to obtain the synthesis gas 4. The organic matter is not particularly limited, and examples thereof include waste plastics, biomass, and organic waste.

The gasification preparation step includes a gasification step and may further include one or more steps of a reforming step, a gas cleaning step, a carbon dioxide separation step, a hydrogenation step, a hydrogen separation step, a shift reaction step, and a reverse shift reaction step.

Here, the gasification preparation step is not particularly limited, a conventionally well-known method can be used, and examples thereof include steps in which a pneumatic type gasifier is used and steps in which an oxygen-steam type gasifier is used. These can be further classified into a fixed bed type and a fluidized bed type, but both types can be used for those steps.

As the reforming step, the same step as described above can be used.

The gas cleaning step is not particularly limited, a conventionally well-known method can be used, and examples thereof include methods in which a bubble stirring tank, a spray tower, a wetted wall tower, or a packed tower is used.

The carbon dioxide separation step is not particularly limited, a conventionally well-known method can be used, and examples thereof include methods in which a carbon dioxide separation apparatus using a pressure swing adsorption method, a temperature swing adsorption method, or a membrane separation method is used.

The hydrogen separation step is not particularly limited, a conventionally well-known method can be used, and examples thereof include methods in which a hydrogen separation apparatus using a pressure swing adsorption method, a temperature swing adsorption method, or a membrane separation method is used.

The shift reaction step and the reverse shift reaction step are not particularly limited, conventionally well-known methods can be used, and the shift reaction step and the reverse shift reaction step can be performed, for example, in the presence of a catalyst. As the catalyst, a conventionally well-known catalyst can be used, there is no particular limitation, and examples thereof include transition metal oxides, such as iron oxide (Fe₃O₄), or platinum. Here, the shift reaction step refers to a step of generating carbon dioxide and hydrogen from carbon monoxide and steam, and the reverse shift reaction step refers to a step of performing a reverse reaction of the shift reaction (generating carbon monoxide and steam from carbon dioxide and hydrogen).

In the hydrogenation step, hydrogen produced outside the system may be used, and in such a case, while not particularly limited, for example, hydrogen obtained using renewable energy is preferably used from the viewpoint of decreasing the amount of carbon dioxide discharged. More specific examples thereof include blue hydrogen, such as by-product hydrogen from an oil refinery facility, by-product hydrogen derived from a chemical process, and by-product hydrogen for which CCS is jointly used, hydrogen from which a gas composition has been adjusted with PSA or the like, water electrolysis hydrogen, salt water electrolysis hydrogen, hydrogen that is obtained by a different electrolysis technique, and hydrogen that is obtained by steam reforming.

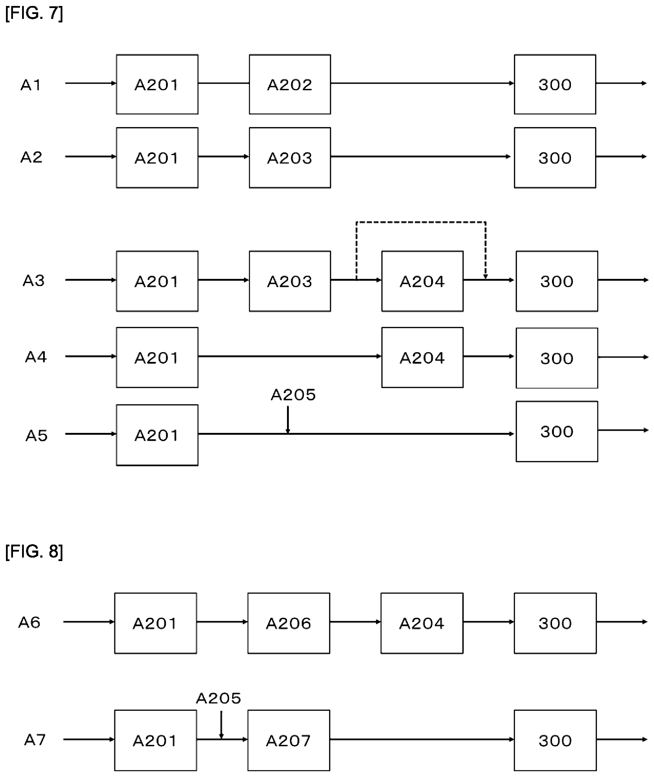

Specific examples of the gasification preparation step are not particularly limited, and, for example, configurations in FIGS. 7 and 8 can be used.

A gasification preparation step A1 in FIG. 7 is a step of obtaining a synthesis gas by undergoing a gasification step A201 and a gas cleaning step A202 in order.

A gasification preparation step A2 in FIG. 7 is a step of obtaining a synthesis gas by undergoing the gasification step A201 and a reforming step A203 in order.

A gasification preparation step A3 in FIG. 7 is a step of obtaining a synthesis gas by undergoing the gasification step A201, the reforming step A203, and a carbon dioxide separation step A204 as necessary in order.

A gasification preparation step A4 in FIG. 7 is a step of obtaining a synthesis gas by undergoing the gasification step A201 and the carbon dioxide separation step A204 in order.

A gasification preparation step A5 in FIG. 7 is a step of obtaining a synthesis gas by undergoing the gasification step A201 and a hydrogenation step A205 in order.

A gasification preparation step A6 in FIG. 8 is a step of obtaining a synthesis gas by undergoing the gasification step A201, a shift reaction step A206, and the carbon dioxide separation step A204 in order.

A gasification preparation step A7 in FIG. 8 is a step of obtaining a synthesis gas by undergoing the gasification step A201, the hydrogenation step A205, and a reverse shift reaction step A207 in order.

### [Step (B)]

The step (B) in the method for producing methanol of the present embodiment is a step of supplying the synthesis gas 4 to a methanol synthesis unit 300 and reacting the synthesis gas in the presence of a catalyst to obtain a crude methanol 5 as shown in FIGS. 1 and 2. A part of a purged gas 9 that is generated as a by-product in association with the generation of the crude methanol may be supplied as the recycled gas 2 to the synthesis gas preparation unit 200 or the desulfurization unit 100 present upstream thereof.

In the step (B), a reaction mixture that is obtained by the reaction is cooled and then separated into gas and liquid, whereby the crude methanol 5 can be obtained as a liquid phase, and the purged gas 9 containing an unreacted gas or the like can be obtained as a gas phase. As a method for the gas-liquid separation, a conventionally well-known method can be used, there is no particular limitation, and for example, a high-pressure separator can be used.

The purged gas means a gas separated from the crude methanol after the methanol synthesis reaction. The purged gas mainly contains a gas or the like that is not used in the reaction. While depending on the conditions of the methanol synthesis reaction, the purged gas is a gas mixture that may contain hydrogen, carbon monoxide, carbon dioxide, methane, nitrogen, and the like as a composition thereof. At least a part of the purged gas is preferably supplied to a shift reaction unit and/or a boiler. This makes it possible to further reduce the amount of carbon dioxide discharged.

The gas temperature at the entry of the methanol synthesis unit 300 is set as appropriate depending on the kind or amount of the catalyst, the shape and reaction pressure of a reactor, and the like, and is preferably 170°C to 260°C, more preferably 170°C to 220°C, and still more preferably 170°C to 200°C. When the entry gas temperature is 170°C or higher, there is a tendency that the reactivity improves, and when the entry gas temperature is 260°C or lower, there is a tendency that the equipment cost can be reduced.

The gas pressure at the entry of the methanol synthesis unit 300 is preferably 4.9 to 14.7 MPaG, more preferably 5.0 to 11.0 MPaG, and still more preferably 5.0 to 10.0 MPaG. When the entry gas pressure is 4.9 MPaG or higher, there is a tendency that the reactivity improves, and when the entry gas pressure is 14.7 MPaG or lower, there is a tendency that the production efficiency increases.

For the synthesis gas 4 that is supplied to the methanol synthesis unit 300, the mol% relationship (M value) among CO, carbon dioxide, and hydrogen that is calculated from the following equation:$M value = \left(hydrogen mol%\right) / \left(2\times CO mol%+3\times carbon dioxide mol%\right)$ is preferably 0.9 to 5.0, more preferably 0.9 to 3.0, still more preferably 0.9 to 2.0, and particularly preferably 1.0 to 1.5. When the M value is 1.3 or more, there is a tendency that the amount of a by-product decreases, and when the M value is 5.0 or less, there is a tendency that the carbon yield is excellent.

Here, the carbon yield means the ratio of the molar flow rate of methanol that is generated in the methanol synthesis unit 300 to the total amount of the molar flow rate of carbon monoxide and the molar flow rate of carbon dioxide that are contained in the reformed gas that is supplied to the methanol synthesis unit 300.

The reaction temperature in the methanol synthesis unit 300 is preferably 200°C to 300°C, more preferably 200°C to 280°C, and still more preferably 200°C to 270°C from the viewpoint of maintaining the reactivity, suppressing a by-product, and protecting the catalyst.

The kind of the methanol synthesis unit 300 is not particularly limited, but is preferably, for example, a unit having a mechanism capable of controlling the reaction temperature. Specific examples thereof include a heat exchange-type reactor and a quench-type adiabatic reactor. The heat exchange-type reactor is not particularly limited, and examples thereof include a multi-tube heat exchange-type reactor and a radial flow-type reactor.

In the case of using the multi-tube heat exchange-type reactor, the reaction temperature is controlled by indirect heat exchange with pressurized boiling water, and saturated vapor (steam) is obtained. The boiling water is circulated in a steam drum and the shell side of the reactor, and the steam is collected from a steam drum. Steam that is obtained with this synthesis system is preferably used as a heat source for the purification process of a methanol solution that is present downstream of a synthesis process. The pressurized boiling water is preferably 220°C to 260°C.

In the case of employing the adiabatic reactor, the reactor has one or more catalyst layers therein, and in a case where the reactor has two or more layers, a part of a synthetic reactor supply gas is branched as a cooling gas of an interlayer and supplied as a quench gas, whereby the reaction temperature is controlled, an evaporator is installed in a reactor exit gas as a heat collector to collect steam, and the steam may be, similarly, used as the heat source for the purification process of the methanol solution downstream.

The catalyst that is used for the synthesis is preferably a methanol synthesis catalyst containing copper atoms and zinc atoms as essential components. Such a catalyst is reduced from an oxide state by a reducing gas, for example, hydrogen, carbon monoxide, or a gas mixture thereof, whereby copper is activated, and the catalyst has a catalyst activity. The catalyst may contain, in addition to the copper atoms and the zinc atoms, aluminum atoms and/or chromium atoms as a third main component. The catalyst containing copper and zinc as essential components can be prepared by a well-known method. Such a catalyst can be prepared by, for example, methods described in Japanese Patent Publication No. 51-44715, Japanese Patent No. 2695663, Japanese Patent Publication No. 6-35401, Japanese Patent Laid-Open No. 10-272361, and Japanese Patent Laid-Open No. 2001-205089.

A preferable catalyst is a methanol synthesis catalyst containing copper atoms and zinc atoms in an atomic ratio (copper/zinc) of 2.0 to 3.0 and containing aluminum atoms. Such a catalyst is not particularly limited, and examples thereof include catalysts prepared by a method described in Japanese Patent Laid-Open No. 8-299796 and a catalyst described in International Publication No. WO 2011/048976.

Specific examples of the preferable catalyst include catalysts used in examples and comparative examples, for example, Example 2 and Example 3, of International Publication No. WO 2011/048976. In addition, a more preferable atomic ratio (copper/zinc) of the copper atoms and the zinc atoms in the catalyst is within a range of 2.1 to 3.0. A methanol synthesis catalyst containing, in addition to those, 3 to 20 mass% of alumina is still more preferable. As described above, such a catalyst is not particularly limited and can be prepared by a method described in International Publication No. WO 2011/048976. More specifically, the catalyst is prepared by, for example, a production method having a step of mixing an aqueous solution containing copper, an aqueous solution containing zinc, and an alkali aqueous solution to generate a precipitate containing copper and zinc, a step of mixing the obtained precipitate and an alumina hydrate having a pseudo-boehmite structure to obtain a mixture, and a step of molding the obtained mixture so that the density reaches 2.0 to 3.0 g/mL. Here, examples of a molding method include tableting, extrusion, and rolling granulation. The catalyst that is used in the present embodiment is not limited to the above-described catalysts and catalysts prepared by the above-described preparation methods and may be a different catalyst having an equivalent methanol synthesis activity.

### [Step (C)]

The method for producing methanol of the present embodiment includes the step (C) of distilling the crude methanol 5 obtained in the step (B) with a distillation unit 400 to separate methanol 10 and distillation wastewater 6, respectively, as shown in FIGS. 1 and 2. The methanol in the present embodiment means purified methanol. Here, the distillation wastewater means a liquid component containing distillation waste liquid (side cut liquid) and may further include distillation wastewater (bottoms). The distillation waste liquid means a liquid component containing concentrated methanol that is drawn out to the outside of a distillation tower in each stage of the tower, concentrated alcohols other than methanol, and water. The distillation wastewater means a liquid component that is drawn out from the tower bottom of the distillation tower to the outside of the tower and is mainly composed of water.

As the distillation unit 400, a conventionally well-known method can be used, there is no particular limitation, and, for example, a distillation tower including a reboiler and a condenser can be used. In such a case, highly pure methanol is obtained from the tower bottom or the tower middle by distilling a methanol mixture.

In the step (C), steam collected in the step (A) or the like may be used. The use of the steam makes it possible to absorb or adsorb a fluid that is discharged from the tower top in the case of, for example, distilling the methanol mixture using a distillation tower. This makes it possible to further reduce the amount of carbon dioxide discharged.

In the step (C), it is also possible to use heat collected in the step (A). Such heat is not particularly limited and can be used as heat necessary in the reboiler in the case of, for example, distilling the methanol mixture using a distillation tower. This makes it possible to further reduce the amount of carbon dioxide discharged. Renewable energy may also be supplied from the outside and used as heat.

### [Step (D)]

The method for producing methanol of the present embodiment includes the step (D) of removing an acid and/or a base and a salt thereof from the distillation wastewater 6 obtained by the step (C) as shown in FIGS. 1 and 2. The step (D) is not particularly limited, and examples thereof include the following first aspect and second aspect.

The first aspect of the step (D) includes a step of removing an acid and/or a base and a salt thereof from the distillation wastewater 6 with a deionization unit 500 as shown in FIG. 1 (hereinafter, also referred to as the deionization step). The distillation wastewater 6 is turned into deionized distillation wastewater 7 with the deionization unit 500.

The deionization unit 500 is not particularly limited, a conventionally well-known unit can be used, and, for example, an ion exchanger, an electric deionization apparatus, and the like can be used. Among them, the ion exchanger is preferable from the viewpoint of the accuracy of deionization, the collection efficiency of a carbon source in the distillation wastewater, and the like. The ion exchanger is not particularly limited, and examples thereof include ion exchange resins and ion exchange films.

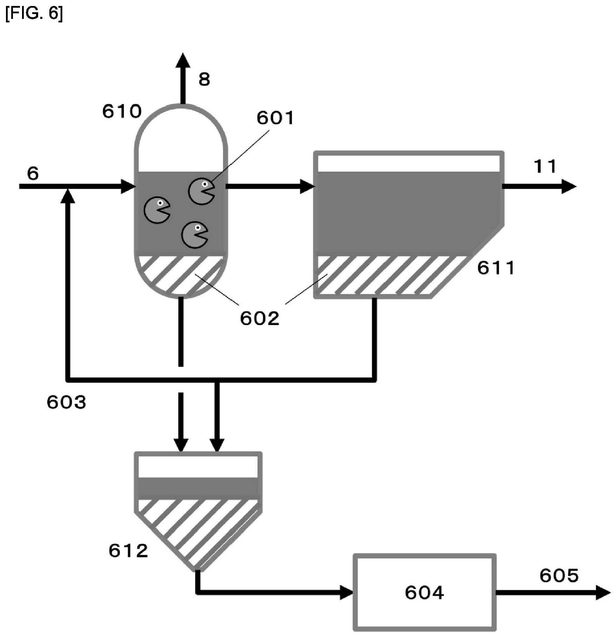

The second aspect of the step (D) includes a step of removing an acid and/or a base and a salt thereof from the distillation wastewater 6 with an anaerobic treatment unit 600 as shown in FIG. 2. The anaerobic treatment unit 600 does not directly deionize the distillation wastewater 6, but is capable of selectively taking out a carbon source or the like in the distillation wastewater 6 and is thereby capable of indirectly performing deionization. Here, the anaerobic treatment refers to a treatment for decomposing an organic matter using a microbe under an atmosphere where oxygen is absent or deficient. The specific configuration of the anaerobic treatment unit is not particularly limited and can be as shown in FIG. 6.

Specifically, in the anaerobic treatment, a digestion gas 8 and treatment water 11 are generated due to the decomposition of the organic matter by the microbe, and it is thus possible to supply the digestion gas 8 to the synthesis gas preparation unit 200 in FIG. 2.

A method for the anaerobic treatment is not particularly limited, a conventionally well-known method can be used, and examples thereof include a method in which an acid formation reaction tank by an acid former is used and a method in which a methane generation reaction tank by a methanogen is used. For example, FIG. 6 shows a method in which a methane generation reaction tank by a methanogen is used, and the distillation wastewater 6 that is injected into the system is first injected into a methane generation reaction tank 610 where a microbe is present. There, the organic matter is decomposed by microbes 601, and the digestion gas 8 is generated. The digestion gas 8 contains methane, carbon dioxide, or the like and can be thus collected and thereby used in the synthesis gas preparation unit 200 as a gas having no influences on the acid and/or the base and the salt thereof that have been contained in the distillation wastewater 6. A part of a solvent in the methane generation reaction tank is sent to a precipitation tank 611 and taken out as the treatment water 11. Returned sludge 603 that is a part of sludge 602 accumulated in the methane generation reaction tank 610 and the precipitation tank 611 is supplied to the methane generation reaction tank 610 again as an organic matter, and the remainder is sent to a sludge concentration tank 612, subjected to a concentration treatment, then, subjected to a dehydration treatment with a sludge dehydration unit 604, and collected as dehydrated sludge 605.

### [Step (H)]

In a case where the step (D) is the anaerobic treatment, the method for producing methanol of the present embodiment preferably further includes the step (H) of combusting a gas that is obtained by the anaerobic treatment to recover heat. Particularly, the step (H) more preferably includes a step of supplying the gas as a fuel for the step (A).

In the method for producing methanol of the present embodiment, the molar flow rate of a carbon atom that is contained in the gas and/or the liquid that is obtained in the step (D) is preferably 70 mol% or more, more preferably 80 mol% or more, still more preferably 90 mol% or more, and particularly preferably 90 mol% or more based on the molar flow rate of a carbon atom that is contained in the distillation wastewater. Such a numerical range is not particularly limited and can be adjusted by, for example, changing the kind of the ion exchanger in the step (D).

### [Step (E)]

The method for producing methanol of the present embodiment includes the step (E) of using the gas and/or the liquid that is obtained in the step (D) in the step (A). Specifically, in a case where the step (D) is the deionization step, the step (E) makes the deionized distillation wastewater 7 obtained by the deionization step be used in the step (A), and in a case where the step (D) is the anaerobic treatment, the step (E) makes the digestion gas 8 that is obtained by the anaerobic treatment be used in the step (A).

### [Step (G)]

The method for producing methanol of the present embodiment preferably further includes the desulfurization step (G). The step (G) is a step of supplying the hydrocarbon-containing gas 1 to the desulfurization unit 100 to obtain the desulfurized gas 3 from which a sulfur content has been removed as shown in FIGS. 1 and 2. In a case where the hydrocarbon-containing gas is a fossil fuel gas, the fossil fuel gas contains a sulfur content, the desulfurization step (G) is thus particularly required. That is, since a sulfur compound becomes a catalyst poison of the catalyst that is used in the step (A) or the step (B), in a case where the hydrocarbon-containing gas 1 contains a sulfur content, it is preferable to remove the sulfur content in advance in the step (G). As a desulfurization method, a conventionally well-known method can be used, there is no particular limitation, and examples thereof include a dry method in which an adsorption agent or a catalyst is used and a wet method in which an amine-based or different absorption liquid is used. In the case of using the dry method, the operation temperature can be set to 0°C to 400°C while varying with the kind of the sulfur compound that is a removal target or the kind of the catalyst that is employed.

### [Other steps]

The method for producing methanol of the present embodiment may include, aside from the above-described steps, other steps as necessary.

### [Apparatus for producing methanol]

An apparatus for producing methanol of the present embodiment is an apparatus for performing the above-described method for producing methanol, and examples thereof include apparatuses shown in configuration views of FIGS. 1 and 2.

The apparatus for producing methanol of the present embodiment is an apparatus for producing methanol including the synthesis gas preparation unit 200, the methanol synthesis unit 300, the distillation unit 400, and the deionization unit 500 or the anaerobic treatment unit 600, in which the gas and/or the liquid that is obtained from the deionization unit 500 or the anaerobic treatment unit 600 is used in the synthesis gas preparation unit 200, as shown in FIG. 1 and FIG. 2. The apparatus for producing methanol of the present embodiment may include other units as necessary.

The synthesis gas preparation unit 200 may include one or more apparatuses or reactors out of a reformer, a gasifier, a gas cleaning apparatus, a carbon dioxide separator, a hydrogen separator, a shift reactor, a reverse shift reactor, and a reforming reactor. Among them, the synthesis gas preparation unit 200 preferably includes a reformer from the viewpoint of improving the carbon yield or the like.

The methanol synthesis unit 300 includes a methanol synthesis reactor where the synthesis gas 4 obtained with the synthesis gas preparation unit 200 is reacted in the presence of a catalyst to generate methanol and an unreacted gas and may include other equipment as necessary.

As the distillation unit 400, a conventionally well-known unit can be used, there is no particular limitation, and examples thereof include distillation towers including a reboiler and a condenser.

The deionization unit 500 is equipment capable of directly removing an ion that is contained in wastewater and is not particularly limited, and examples thereof include apparatuses in which an ion exchanger is used and electric deionization apparatuses. The anaerobic treatment unit 600 is not particularly limited as long as the apparatus has a decomposition tank by anaerobic microbe.

The apparatus for producing methanol of the present embodiment preferably further includes the desulfurization unit 100 as shown in FIGS. 1 and 2.

The desulfurization unit 100 includes a desulfurizer where a sulfur content is removed from the hydrocarbon-containing gas and may include different equipment as necessary.

### Examples

Hereinafter, the method for producing methanol and apparatus for producing methanol of the present invention will be described in detail with examples and comparative examples, but the present invention is not limited thereto.

As a catalyst that was used for methanol synthesis, any of a catalyst prepared by a method described in Example 1 of Japanese Patent Publication No. 51-44715 (methanol synthesis catalyst A), a catalyst prepared by a method described in Example 1 of Japanese Patent Laid-Open No. 8-299796 (methanol synthesis catalyst B), a catalyst prepared by a method described in Example 3 of International Publication No. WO 2011/048976 (methanol synthesis catalyst C), or a catalyst prepared by a method described in Comparative Example 4 of Japanese Patent Laid-Open No. 8-299796 (methanol synthesis catalyst D) was used. The amount of the catalyst used in each of the examples and the comparative examples below was set to the same amount.

### [Example 1]

In Example 1, a production apparatus shown in FIG. 1 was used. Each condition was as shown in Table 1. That is, a shell gas (CH₄: 94.3 mol%, C₂H₆: 2.7 mol%, C₃H₈: 0.6 mol%, C₄H₁₀: 0.2 mol%, C₅H₁₂: 0.2 mol%, carbon dioxide: 0.5 mol%, and N₂: 1.5 mol%) was used as a hydrocarbon-containing gas 1, a steam reforming reaction was performed, and methanol was then synthesized using a synthetic gas that was generated by the steam reforming reaction. The methanol synthesis catalyst C was used as a catalyst in a methanol synthesis reactor in a methanol synthesis unit. A multi-tube heat exchange-type reactor was used as the methanol synthesis reactor. Regarding set conditions, the pressure of a fluid that was to come into contact with the catalyst in the reactor was set to 10.0 MPaG, the shell pressure was set to 4.0 MPaG, and the temperature was 200°C to 234°C. The reaction pressure in a reformer was 1.9 MPaG, and the temperature was 860°C. Distillation was performed under conditions of a methanol distillation efficiency of 99% and a tower top composition of ethanol of 5 ppm. The same ion exchanger/apparatus as that described in Japanese Patent No. 3468259 was used as a deionization unit 500.

In Example 2, the same operation as in FIG. 1 was performed except that the deionization unit 500 was replaced by an anaerobic treatment unit 600, and a digestion gas obtained from the anaerobic treatment unit 600 was supplied to a synthesis gas preparation unit 200 as shown in FIG. 2. The results of the amount of methanol produced (ton/D) by Example 2 and the like are shown in Table 1. The same apparatus as in Japanese Patent Publication No. 58-33040 was used as the anaerobic treatment unit 600.

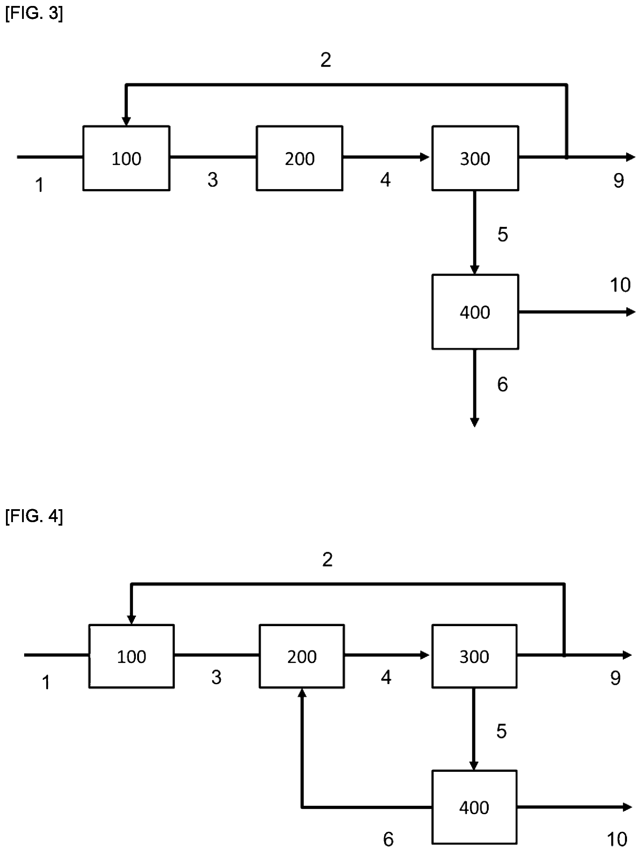

In Comparative Example 1, the same operation as in Example 1 was performed except that the deionization unit 500 was not provided, and no distillation wastewater was supplied to a synthesis gas preparation unit 200 as shown in FIG. 3. The results of the amount of methanol produced (ton/D) by Comparative Example 1 and the like are shown in Table 1.

In Comparative Example 2, the same operation as in Example 1 was performed except that the deionization unit 500 was not provided as shown in FIG. 4. The results of the amount of methanol produced (ton/D) by Comparative Example 2 and the like are shown in Table 1.

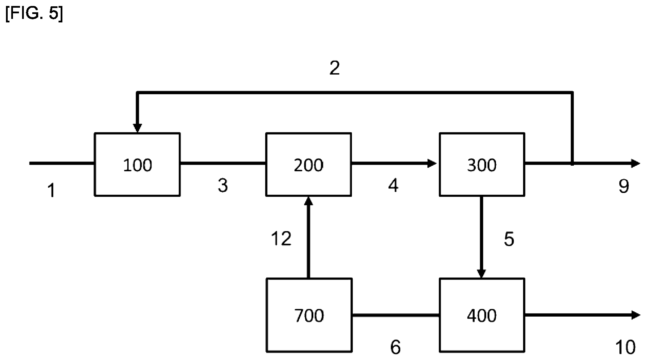

In Comparative Example 3, the same operation as in Example 1 was performed except that the deionization unit 500 was replaced by a neutralization unit 700 as shown in FIG. 5. The results of the amount of methanol produced (ton/D) by Comparative Example 3 and the like are shown in Table 1.

**[Table 1]**

| | Unit | Example 1 | Example 2 | Comparative Example 1 | Comparative Example 2 | Comparative Example 3 |
|---|---|---|---|---|---|---|
| Amount of carbon dioxide that is absorbed into alkali in distillation wastewater into methanol | kg/D | 0 | 0 | 89 | 89 | 89 |
| Amount of methanol produced | ton/D | 2508.7 | 2506.1 | 2500 | 2508.6 | 2508.6 |
| pH of distillation wastewater | - | 12 | 12 | 12 | 12 | 12 |
| pH of deionized distillation wastewater | - | 7 | - | - | 12 | 7 |
| Salt concentration in distillation wastewater | wt% | 0.05 | 0.05 | 0.05 | 0.05 | 0.05 |
| Salt concentration in deionized distillation wastewater | wt% | 0 | - | - | 0.05 | 0.05 |

What has been described above showed that, in Examples 1 and 2, it was possible to recycle a component in distillation wastewater while realizing a pH and a salt concentration at which the distillation wastewater did not damage the equipment.

### Industrial Applicability

The present invention is industrially applicable in methods and apparatuses for producing methanol.

The present application claims priority based on Japanese Patent Application (Japanese Patent Application No. 2023-218417), filed on December 25, 2023, the content of which is incorporated thereinto by reference.

### Reference Signs List

1 ··· Hydrocarbon-containing gas, 2 ··· recycled gas, 3 ··· desulfurized gas, 4 ··· synthesis gas, 5 ··· crude methanol, 6 ··· distillation wastewater, 7 ··· deionized distillation wastewater, 8 ··· digestion gas, 9 ··· purged gas, 10 ··· methanol, 11 ··· treatment water, 12 ··· neutralized distillation wastewater, 200 ··· synthesis gas preparation unit, 300 ··· methanol synthesis unit, 400 ··· distillation unit, 500 ··· deionization unit, 600 ··· anaerobic treatment unit, 601 ··· microbe, 602 ··· sludge, 603 ··· returned sludge, 604 sludge dehydration unit, 605 dehydrated sludge, 610 methane generation reaction tank, 611 ··· precipitation tank, 612 ··· sludge concentration tank, 700 ··· neutralization unit, A1 to A8 ··· gasification preparation step, A201 ··· gasification unit, A202 ··· gas cleaning step, A203 ··· reforming step, A204 ··· carbon dioxide separation step, A205 ··· hydrogenation step, A206 ··· shift reaction step, A207 ··· reverse shift reaction step, A208 ··· hydrogen separation step

## Claims

1. A method for producing methanol, comprising:
a step (A) of obtaining a synthesis gas containing at least carbon dioxide and hydrogen;
a step (B) of reacting the synthesis gas in the presence of a catalyst to obtain a methanol mixture;
a step (C) of distilling the methanol mixture to separate methanol and distillation wastewater, respectively;
a step (D) of removing an acid and/or a base and a salt thereof from the distillation wastewater; and
a step (E) of feeding a gas and/or a liquid that is obtained in the step (D) to the step (A).

2. The method for producing methanol according to Claim 1, wherein a molar flow rate of a carbon atom that is contained in the gas and/or the liquid that is obtained in the step (D) is 70 mol% or more based on a molar flow rate of a carbon atom that is contained in the distillation wastewater.

3. The method for producing methanol according to Claim 1 or 2, wherein the step (A) comprises a step (F) of reforming a hydrocarbon that is contained in the gas and/or the liquid that is obtained in the step (D) to obtain a reformed gas.

4. The method for producing methanol according to Claim 1 or 2, further comprising:
a desulfurization step (G).

5. The method for producing methanol according to Claim 1 or 2, wherein the step (D) comprises a step of removing the acid and/or the base and the salt thereof with an ion exchanger.

6. The method for producing methanol according to Claim 1 or 2, wherein the step (D) comprises a step of removing the acid and/or the base and the salt thereof by an anaerobic treatment.

7. The method for producing methanol according to Claim 6, further comprising:
a step (H) of combusting a gas that is obtained by the anaerobic treatment to recover heat.

8. An apparatus for producing methanol, comprising:
a synthesis gas preparation unit;
a methanol synthesis unit;
a distillation unit; and
a deionization unit or an anaerobic treatment unit,
wherein a gas and/or a liquid that is obtained from the deionization unit or the anaerobic treatment unit is used in the synthesis gas preparation unit.

9. The apparatus for producing methanol according to Claim 8, wherein the synthesis gas preparation unit comprises a reforming apparatus.

10. The apparatus for producing methanol according to Claim 8 or 9, further comprising:
a desulfurization unit.
